# EUROPEAN PATENT SPECIFICATION

(11) **EP 2 077 852 B1**
(45) Date of publication and mention of the grant of the patent: **27.11.2013**
(21) Application number: 07833661.7
(22) Date of filing: 29.10.2007
(51) Int. Cl.: A61K 38/19, A61P 25/02

(54) **AGENT COMPRISING G-CSF FOR PREVENTION AND TREATMENT OF DIABETIC PERIPHERAL NEUROPATHY**
MITTEL MIT G-CSF ZUR PRÄVENTION UND BEHANDLUNG VON DIABETISCHER PERIPHERER NEUROPATHIE
AGENT CONTENANT DU G-CSF DESTINÉ À PRÉVENIR ET À TRAITER LA NEUROPATHIE PÉRIPHÉRIQUE DIABÉTIQUE

(30) Priority: 30.10.2006 KR 20060105684
(43) Date of publication of application: 15.07.2009
(73) Proprietor: DONG-A PHARM. CO., LTD., Seoul 130-708 (KR)
(72) Inventor: KIM, Kyung-Soo, Seoul 133-791 (KR); JIN, Ji-Yong, Seoul 133-791 (KR)
(74) Representative: Albrecht, Thomas
(86) International application number: PCT/KR2007/005353
(87) International publication number: WO 2008/054098

(56) References cited:
- WO-A1-2004/091661
- TAM J ET AL: "Dual-action peptides: a new strategy in the treatment of diabetes-associated neuropathy", DRUG DISCOVERY TODAY, ELSEVIER, RAHWAY, NJ, US, vol. 11, no. 5-6, 1 March 2006 (2006-03-01), pages 254-260, XP025027079, ISSN: 1359-6446, DOI: 10.1016/S1359-6446(05)03722-0 [retrieved on 2006-03-01]
- AL-BAHAR S ET AL: "Granulocyte colony-stimulating factor potentiated severe atypical vincristine-induced neuropathy", MEDICAL PRINCIPLES AND PRACTICE 1998 CH LNKD- DOI:10.1159/000026024, vol. 7, no. 1, 1998, pages 68-73, XP002665486, ISSN: 1011-7571
- "The potential application of granulocyte colony stimulating factor therapy on neuropathic pain ny", , 1 May 2009 (2009-05-01), XP55014460, Retrieved from the Internet: URL:http://memo.cgu.edu.tw/cgmj/3203/32030 1.pdf [retrieved on 2011-12-09]
- JUNG K.H. ET AL.: 'Granulocyte-to-colony-stimulating factor stimulates neurogenesis via vascular endothelial growth factor with STAT activation' BRAIN RESEARCH 09 January 2006, pages 190 - 201, XP025064587
- HUANG P. ET AL.: 'Autologous Transplantation of Granulocyte Colony-Stimulating Factor-Mobilized Peripheral Blood Mononuclear Cells Improves Critical Limb Ischemia in Diabetes' DIABETES CARE vol. 28, September 2005, pages 2155 - 2160, XP008106112

## Description

### Technical Field

The present invention relates to an agent for preventing and treating diabetic peripheral neuropathy including a granulocyte colony stimulating factor (G-CSF) as an active ingredient.

### Background Art

Diabetes is the leading known adult disease in the world, and recently in Korea, its prevalence has reached 7 to 10% with the rapid economical growth. Moreover, the disease has become the leading cause of death for the people in the age of 60's and 70's.

Diabetes is a syndrome generated by being unable to secrete insulin that functions to carry glucose into the cells or the insulin being unable to function well such that the glucose is not transferred into the cells. Under circumstances, the glucose, which has not transferred into the cells, remains in blood. Thus, the blood becomes hyperglycemia, and the glucose is secreted out via urine. Continuation of the hyperglycemia results in disordered metabolism of proteins and lipids in the body and causes physiological and biochemical problems in the organisms, thereby inducing complications.

Diabetic peripheral neuropathy is one of the three major complications of diabetes together with diabetic retinopathy and diabetic nephropathy. The disease may not have any symptoms, but may develop serious pain or loss of sensation to the legs, muscle weakness, or autonomic neuropathy. And its treatment is very difficult.

The prevalence of the diabetic peripheral neuropathy is 5 to 60%, which shows big difference depending on the researchers, and in the patients with diabetes is approximately 12%. After 25 years with diabetes, the prevalence is reported to be approximately 60%.

The usual symptoms of the diabetic peripheral neuropathy are expressed as paraesthesia, such as numbness of hands and feet, usually feet, burning pain, pricking pain, sensation as if stepping on sand, or floor being covered with something, sensory loss, cold feet even in summers.

In the early stage of the disease, only the symptoms of paraesthesia by the sensory nerve stimulation, without the change of skin sensory, may be observed. However, when the sensory nerve is further damaged with the progression of the disease, the skin in the region controlled by the sensory nerve may develop sense of temperature, pain, vibration or touch.

Diabetic peripheral neuropathy is caused by high blood sugar such that biochemical and conformational change (axonal atrophy, nodular swelling, microvascular change, etc.) are occurred inside the nerve cells. However, details thereof are not certain.

As a method for the treatment of such diabetic peripheral neuropathy, nerve block therapy, metabolism control and pharmacotherapy (see Tam et al. Drug Discovery Today, vol. 11, N° 5-6, pp 254-260), are mentioned. However, among the pharmacotherapy, no one drug is effective for the significant improvement in the symptom as well as the substantial cure.

Therefore, researches on a mechanism of diabetic peripheral neuropathy and a substantial development of drugs based on the researches are in desperate need.

Meanwhile, a granulocyte-colony stimulating factor (G-CSF) has specific functions on neutrophil progenitor cells and it promotes neutrophil proliferation and differentiation, and increases antibody dependent cellular cytotoxicity of neurophil. Moreover, the G-CSF also has functions to induce IgA-mediated phagocytosis and increase superoxide production ability.

Therefore, the G-CSF improves response to chemotactic peptide which is known to play a role in the inhibition of infection and reduction in fever. Furthermore, the G-CSF has functions on more differentiated myeloid cells compared with the other CSFs such as a granulocyte-macrophage CSF (GM-CSF). Thus, it is expected to have less effect on blast-cells in patients with leukemia *in vivo.*

Accordingly, the G-CSF is widely used as a drug for inducing neutrophil in anticancer chemotherapy, anticancer drug megadose therapy, combination therapy with radiotherapy, and after the bone-marrow transplantation (Julie M. Vores et al., Clinical Applications of Hematopoietic Growth Factors, Journal of Clinical Oncology, 13 : 1023-1035, 1995).

The G-CSF is mostly used as a hematopoietic agent, which mainly functions in proliferation and differentiation of neutrophils, in neutropenia caused by bone-marrow transplantation and anticancer drug administration. Also, it is used in increasing neutrophils in the patients with serious chronic neutropenia such as myelodysplastic syndromes, aplastic anemia, or congenital, cyclic, idiopathic neutropenia, and HIV infection, and in preventing infections caused by neutropenia.

Neutrophils are phagocytic cells having an important role in host defense mechanism. In the case of normal immune function and hematopoietic status, the number of neutrophil increases during infection. The state, in which the neutrophil count is reduced to 1500 cells/mm² or less, is referred to as neutropenia. When the neutrophil count is 500 cells/mm² or less, a normal host defense mechanism is greatly damaged such that it largely increases the danger of bacterial infection.

Recently, in addition to using the above-mentioned G-CSF clinically in neutropenia, with expectations that the G-CSF is effective in the prevention and treatment of various infectious diseases such as pneumonia and sepsis by enhancing functions to induce neutrophil production, researches on a single administration of the G-CSF or co-administration with antibiotics to the infectious diseases are in progress.

Matured G-CSF protein is composed of 4 alpha-helixes and has 2 disulfide bonds with a molecular weight of about 20,000, in which the threonine substituent at 133-position is the only position where O-linked carbohydrate is being added. G-CSF receptors existing on the surfaces of granulocytes has a molecular weight of about 150,000, is composed of a single peptide chain, and is N-glycosylated. With the maturation of the cells, the number of receptors increases such that it becomes several hundreds per cell.

As a drug using G-CSF, Korean Patent Application No. 10-2005-7019543 (Publication No. 2005-0114275) discloses an agent comprising at least one stem cell recruitment factor such as a G-CSF as an active ingredient for the treatment of diabetes.

Korean Patent Application NO. 10-2004-7007275 (Publication No. 2005-0044444) discloses a drug comprising cytokine selected from the group consisting of a cytokine for activating a monocyte or macrophage, a cytokine secreted from the activated monocyte or macrophage, and a cytokine secreted from hemopoietic cells that expresses G-CSF receptors, as an active ingredient for mobilizing pluripotent stem cells from tissue into peripheral blood.

However, researches on a novel use of G-CSF as a treatment for diabetic peripheral neuropathy have yet conducted.

### Disclosure

### Technical Problem

It is another object of the present invention to provide an agent for regenerating of peripheral nerves comprising a granulocyte colony stimulating factor (G-CSF) as an active ingredient.

### Technical Solution

In accordance with an aspect of the present invention, the above and other objects can be accomplished by the provision of an agent for preventing and treating diabetic peripheral neuropathy comprising a granulocyte colony stimulating factor (G-CSF) as an active ingredient.

In accordance with another aspect of the present invention, there is provided an agent for regenerating peripheral nerves comprising a granulocyte colony stimulating factor (G-CSF) as an active ingredient.

### Advantageous Effect

In the present invention, the G-CSF regenerates blood vessels in peripheral nerve tissues and rehabilitates damaged nerve tissues, thereby having effects on increasing nerve conduction velocity and improving pain sensitivity. Therefore, the present invention can be a useful agent for preventing and treating diabetic peripheral neuropathy.

### Description of Drawings

The above and other objects, features and other advantages of the present invention will be more clearly understood from the following detailed description taken in conjunction with the accompanying drawings, in which:
FIG. 1 is a graph illustrating nerve conduction velocity measured before and after the administration of a G-CSF (G-CSF administration group) and saline (control group);
FIG. 2 is a graph illustrating pain sensitivity measured before and after the administration of a G-CSF (G-CSF administration group) and saline (control group);
FIG. 3 is a photograph of toluidine blue staining on tail-nerves of rats in Control Group (saline administration);
FIG. 4 is a photograph of toluidine blue staining on tail-nerves of rats in the G-CSF administration group;
FIG. 5 is a transmission electron micrograph (TEM) of tail-nerves of rats in Control Group (saline administration);
FIG. 6 is a transmission electron micrograph (TEM) of tail-nerves of rats in the G-CSF administration group; and
FIG. 7 is a graph illustrating the results from the evaluation with Toronto Clinical Neuropathy Scoring System on the degree of diabetic peripheral neuropathy of the subject patients, 1 week, 15 days, 1 month, 2 months, and 3 months after the administration of G-CSF.

### Best mode

The present invention will now be described in greater detail.

The present inventors have conducted researches on various physiological activities on a granulocyte colony stimulating factor (hereinafter, referred to as 'G-CSF'), and as a result, they have found that the G-CSF regenerates blood vessels in peripheral nerve tissues and rehabilitates damaged nerve tissues, thereby improving nerve conduction velocity and pain sensitivity. Thus, the G-CSF of the present invention can be a useful agent for preventing and treating diabetic peripheral neuropathy. The present invention has been completed based on these findings.

The treating agent of the present invention contains a G-CSF as an active ingredient. Moreover, the agent is characterized by the regeneration of peripheral nerves.

When the agent of the present invention was administered to a type II diabetic animal model Otsuka Long Evans Tokushima Fatty (OLETF) rat, nerve conduction velocity increased and pain sensitivity improved. Further, when the agent of the present invention was administered to a subject patient in the clinical test and evaluated for diabetic peripheral neuropathy with Toronto Clinical Neuropathy Scoring System, the degree of the diabetic peripheral neuropathy improved and the nerve conduction velocity increased.

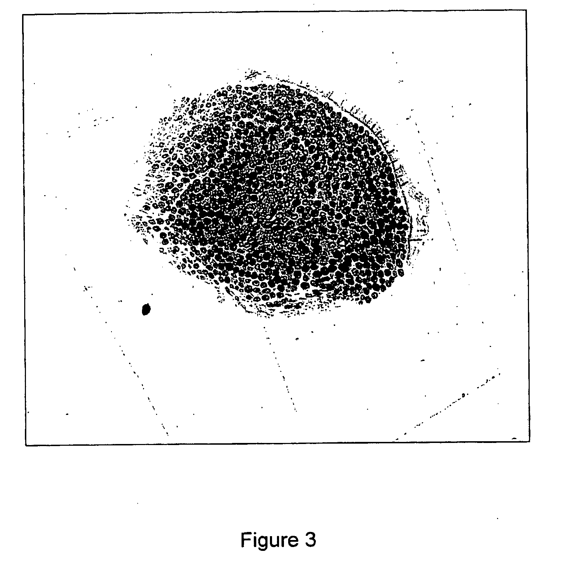

In the animal test using the type II diabetic animal model OLETF rat, regenerated nerve fibers and remyelinated axons as evidences of the peripheral nerve regeneration from the tail nerve of the G-CSF administration group could be observed (see FIGs. 3, 4, 5, and 6).

This is thought that the G-CSF regenerates peripheral nerves and treats diabetic peripheral neuropathy by discharging functional stem cells from bone-marrow into peripheral blood, and inducing the discharged cell differentiation to regenerate nerve cells and blood vessels at the peripheral nerve tissue and recover the damaged nerve tissues, and smoothly supplying blood to nerves.

In general, the G-CSF that can be used in the present invention is preferably a natural G-CSF or recombination G-CSF. Moreover, the G-CSF that can be used in the present invention is the G-CSF having the same amino acid sequence as the natural G-CSF.

The G-CSF of the present invention may be prepared by separating from a mammary organism, synthesizing chemically, or genetically expressing exogenous DNA sequence obtained by genome or cDNA cloning or DNA synthesis in a prokaryotic or eukaryotic host cell.

At this time, a suitable prokaryotic host includes various bacteria (e.g., *E*. *coli*) and a suitable eukaryotic host includes yeast (e.g., *S*. *Serevisiae*) and mammary cells (e.g., ovary cells of a Chinese hamster, cells of a monkey).

Although the recombination G-CSF, particularly a G-CSF derived from *E*. *coli,* is preferable from a maximum commercial point of view, the present invention includes the use of an arbitrary G-CSF among the above G-CSF forms or all G-CSFs. Here, G-CSFs and analogs thereof can be used by obtaining from a variety of suppliers and purifying the same. Most preferably, a recombinant human granulocyte colony-stimulating factor (rhG-CSF) is used.

The therapeutic agent of the present invention comprising the G-CSF as an active ingredient may contain the active ingredient in an amount of 0.0001 to 50% by weight based on the total weight of the therapeutic agent composition.

Moreover, the therapeutic agent of the present invention may further include at least one active ingredient that has the same or similar function with the above-mentioned active ingredient, in addition to the active ingredient.

The therapeutic agent of the present invention comprising the G-CSF as an active ingredient may further include at least one pharmaceutically acceptable carrier in addition to the above-mentioned active ingredient to preferably prepare a pharmaceutical composition.

In preparing the composition in a liquid solution, as the pharmaceutically acceptable carrier, which is suitable for sterilization and *in vivo,* the preferable liquid solution can be selected from the group consisting of saline, sterilized water, Ringer's solution, buffer saline, a albumin injection solution, a dextrose solution, a maltodextrin solution, glycerol, ethanol, or a mixture thereof. If necessary, the composition may include other typical additives such as an antioxidant, a buffer, or a bacteriostatic agent.

Further, a diluent, a dispersant, a surfactant, a binder, or a lubricant may be additionally added to prepare the composition into a form of injections such as a solution, a suspension or an emulsion, pills, a capsules, granules, or tablets. The composition may be used by bonding a site specific antibody or other ligand with the carrier such that the composition has a site specific function. Furthermore, in an appropriate method in this field of art, the composition may be preferably prepared in accordance with each disease or component using a method disclosed in Remington's Pharmaceutical Science, Mack Publishing Company, Easton PA.

A pharmaceutical form of the therapeutic agent of the present invention comprising the G-CSF as an active ingredient may be in granules, powders, coated tablets, capsules, suppositories, syrups, juices, suspensions, emulsions, drops, injectable solutions, and also preparations with sustained release of active compound.

The therapeutic agent of the present invention comprising the G-CSF as an active ingredient may be administered in a typical method through an intravenous, intra-arterial, intraperitoneal, intrasternal, intradermal, nasal, inhalant, topical, rectal, oral, intraocular or subcutaneous route. The administration method is not particularly limited thereto, but a non-oral administration is preferable, and the subcutaneous administration is more preferable.

Dosages of the therapeutic agent of the present invention may be adjusted depending on various factors such as a type of disease, a degree of illness, a type and content of an active ingredient and other components contained in a composition, a type of pharmaceutical form, a patient's age, weight, general health status, gender and dietary, an administration time, an administration route, a flow rate of a composition, a treatment duration, and other drugs used simultaneously. In case of an adult, when the G-CSF is administered once daily for continuous 1 day to 1 week, it may be administered in a dose of 0.01 µg/kg/day to 100 µg/kg/day, and preferably 0.01 µg/kg/day to 10 µg/kg/day.

The therapeutic agent of the present invention may be used alone or in combinations with nerve block therapy, metabolism control, or the like.

The therapeutic agent of the present invention recovers damaged nerve tissues by regenerating nerve cells and blood vessels in peripheral nerve tissues and smoothly supplies blood to the nerve tissues, thereby improving nerve conduction velocity and pain sensitivity.

Accordingly, the therapeutic agent of the present invention recovers damaged nerve tissues by regenerating nerve cells and blood vessels in peripheral nerve tissues and smoothly supplies blood to the nerve tissues, thereby improving nerve conduction velocity and pain sensitivity. Thus, the agent can be useful for preventing and treating diabetic peripheral neuropathy.

### Mode for Invention

Preferred Examples of the present invention and Comparative Examples will now be described. The following Examples and Comparative Examples are provided for illustrative purposes only and are in no way intended to limit the scope of the present invention.

### Examples

Now, the present invention will be described in more detail with reference to the following Examples. These examples are provided only for illustrating the present invention and should not be construed as limiting the scope and spirit of the present invention.

### [Examples]

### Example 1: Animal Test for Confirming Therapeutic Effect of G-CSF on Diabetic Peripheral Neuropathy

An animal model for diabetic peripheral neuropathy was made using a method similar to the method described in the literature [Nakamura J et al., Diabetes Research Clinical Practice, 2001 Jan; 51(1):9-20].

That is, genetically manipulated type II diabetic animal model OLETF rats were bred at a place with good lighting and airing while maintaining 20 to 24°C and a humidity of 40 to 70%. During the breeding, plain solid laboratory chow and tap water were supplied freely. After about 10 weeks, 30 w/v% sugar in water was administered instead of tap water. The total administration period of sugar water was 24 weeks, and weight and blood sugar were measured every 5 weeks. The weight and blood sugar of the G-CSF administration group and the control group at about 34 weeks were measured, and the results are presented in Table 1.

OLETF rats at about 34 weeks were classified into the G-CSF administration group and the control group. In the case of the G-CSF administration group, a G-CSF (Leucostim, manufactured by Dong-A PHARM. Co., LTD) of 100 µg/kg/day was injected to abdominal subcutis once a day for 5 continuous days. Meanwhile, in the case of the control group, saline of 0.2 ml was injected to abdominal subcutis once a day for 5 continuous days.

Before the administration of G-CSF, the difference in nerve conduction velocity between the two groups was measured through the tail nerve. Then, 4 weeks after the G-CSF administration, the nerve conduction velocity and pain sensitivity were measured.

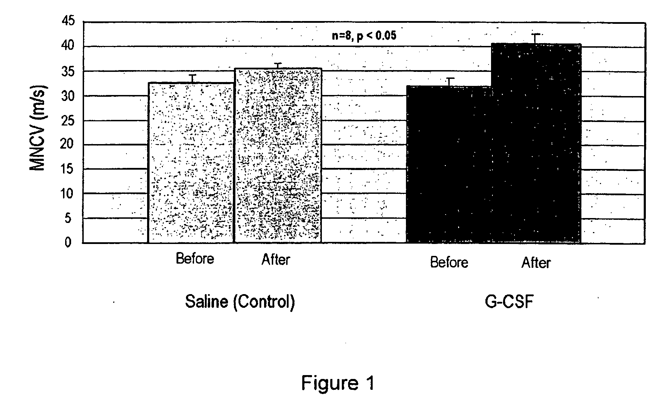

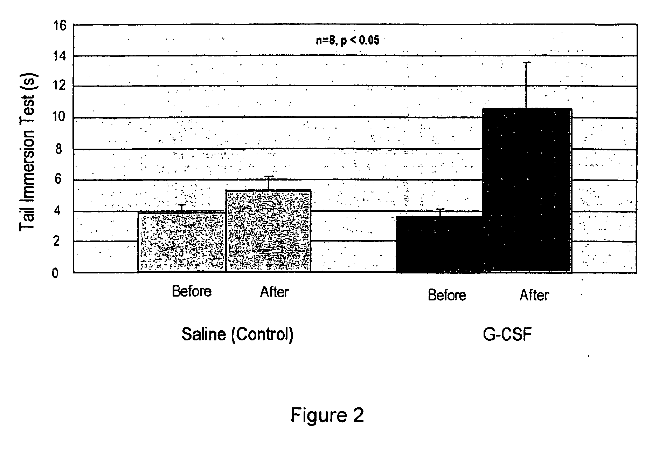

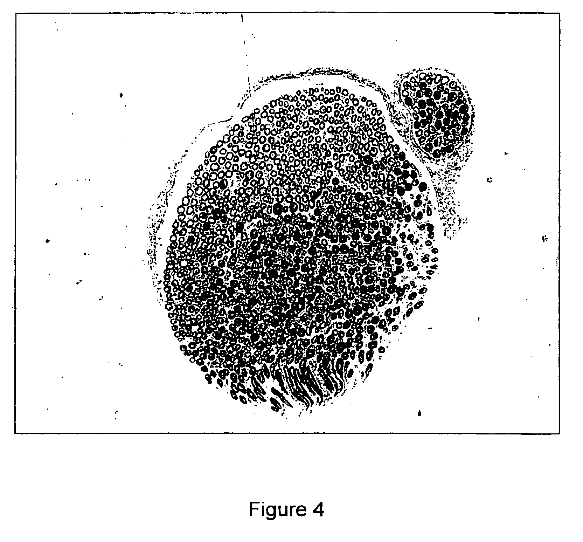

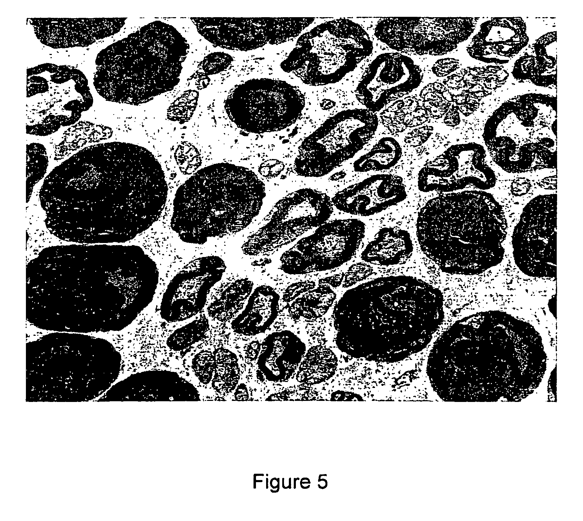

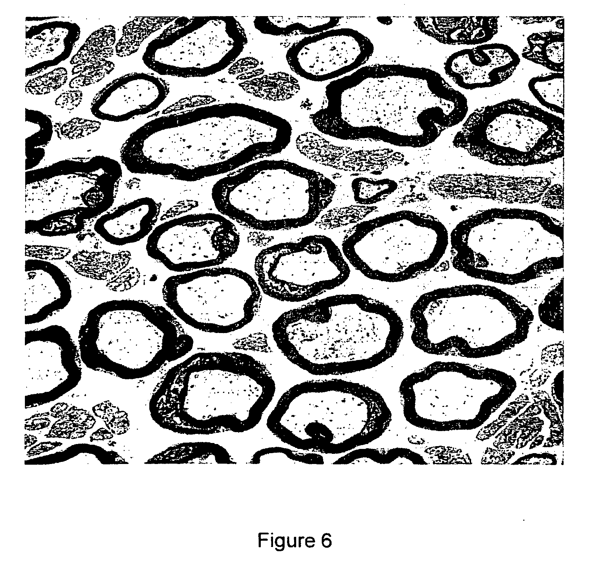

The results are presented in the following Tables 2 and 3, and FIGs. 1 and 2. 4 Weeks after the drug administration, the rats' tail nerves of the G-CSF administration group and control group were extracted. The nerves were stained with toluidine blue and observed with a microscope as shown in FIGs. 3 and 4. The rats' tail nerve tissues of the G-CSF administration group and control group were extracted. The tissues were immunostained and observed the fine structural change with an electron microscope as shown in FIGs. 5 and 6.

**[Table 1]**

| Weight and Blood Sugar of OLETF Rats at 34^{th} weeks | | | |
|---|---|---|---|
| Classification | n (Number of Animals) | Weight (g) | Blood Sugar (mg/dl) |
| Test Group (G-CSF Administration) | 8 | 407 | 589 |
| | | 492 | 500 |
| | | 461 | 422 |
| | | 436 | 411 |
| | | 543 | 504 |
| | | 503 | 482 |
| | | 475 | 448 |
| | | 355 | 469 |
| Control Group (Saline Administration) | 8 | 460 | 429 |
| | | 452 | 445 |
| | | 418 | 463 |
| | | 480 | 538 |
| | | 449 | 456 |
| | | 440 | 426 |
| | | 438 | 560 |
| | | 479 | 528 |

**[Table 2]**

| Nerve Conduction Velocity (m/s) | | |
|---|---|---|
| Classification | Day 0 | Day 28 |
| Control Group (Saline Administration) | 32.63 ±1.6 m/s | 35.58 ±0.9 m/s |
| Test Group (G-CSF Administration) | 31.93 ±1.5 m/s | 40.68 ±1.9 m/s |

**[Table 3]**

| Pain Sensitivity (s) | | |
|---|---|---|
| Classification | Day 0 | Day 28 |
| Control Group (Saline Administration) | 3.6 ±0.8 s | 5.3 ±0.4 s |
| Test Group (G-CSF Administration) | 3.8 ±0.5 s | 10.5 ±2.9 s |

As seen from Table 2 and FIG. 1, it was confirmed that the G-CSF administration group exhibited increased nerve conduction velocity after the G-CSF administration, and also confirmed that the G-CSF administration group exhibited increased nerve conduction velocity compared with the control group of saline administration.

As seen from Table 3 and FIG. 2, it was confirmed that the G-CSF administration group exhibited improved pain sensitivity after the G-CSF administration, and also confirmed that the G-CSF administration group exhibited improved pain sensitivity compared with the control group of saline administration.

Further, as seen from FIGs. 3 and 4, it was observed that the photograph of toluidine blue stained tail nerves of the control group rats in FIG. 3 shows more damaged nerve fibers compared with regenerated nerve fibers, while the photograph of toluidine blue stained tail nerves of the G-CSF administration group rats in FIG. 4 shows far more regenerated nerve fibers compared with damaged nerve fibers.

As seen from FIGs. 5 and 6, it was observed that the TEM of tail nerves of the control group rats in FIG. 5 shows demyelinated axons and destroyed myelin, while the TEM of tail nerves of the G-CSF administration group rats in FIG. 6 shows remyelinated axons which are an evidence of nerve regeneration.

### Example 2: Clinical Test for Confirming Therapeutic Effect of G-CSF on Diabetic Peripheral Neuropathy

Clinical tests have been conducted on patients with diabetic peripheral neuropathy to understand the therapeutic effects of the present invention on diabetic peripheral neuropathy.

1. Five patients were selected from the department of internal medicine (Endocrinology), and basic tests (HOMA, HgA1C, C-peptide, Retinopathy, microalbuminuria for 24 hours, cystatine C and endocrine tests) on diabetes have been conducted.

Respective gender, age, height, weight (kg), blood sugar, and diagnosed disease of the five patients to be treated are listed in Table 4.

**[Table 4]**

| Classification | Gender | Age | Height | Weight | Blood Sugar | Diagnosed Disease |
|---|---|---|---|---|---|---|
| case 1 | M | 78 | 165 | 60 | 132 | Diabetic Peripheral Neuropathy, Coronary Artery Disease (CAD) |
| case 2 | F | 59 | 168 | 79 | 102 | Diabetic Peripheral Neuropathy, Acute Myocardial Infarction (AMI) |
| case 3 | M | 65 | 166 | 66 | 78 | Diabetic Peripheral Neuropathy, Acute Myocardial Infarction (AMI) |
| case 4 | F | 61 | 160 | 65 | 161 | Diabetic Peripheral Neuropathy, Acute Myocardial Infarction (AMI) |
| case 5 | M | 65 | 164 | 64 | 141 | Diabetic Peripheral Neuropathy, Acute Myocardial Infarction (AMI) |

Further, special tests such as neurological scoring and nerve conduction velocity (NCV) measurement in all test groups have been conducted.

The patients were hospitalized 3 days before the administration of G-CSF, and macrovascular complication (CAG, IMT, and ECHO) and other vascular related tests have been conducted on the patients. Next, 1 day before the administration of G-CSF, the patients were transferred to hemato-oncology for testing hematological tests.

Subsequently, 10 µg/kg of a G-CSF (Leucostim, manufactured by Dong-A PHARM. Co., LTD) was injected to subcutis once daily for continuous 4 days to the patients to be treated. After the administration of G-CSF, hematological tests were observed. If no side effects have been occurred, the patients were discharged from the hospital performing only the neurological tests.

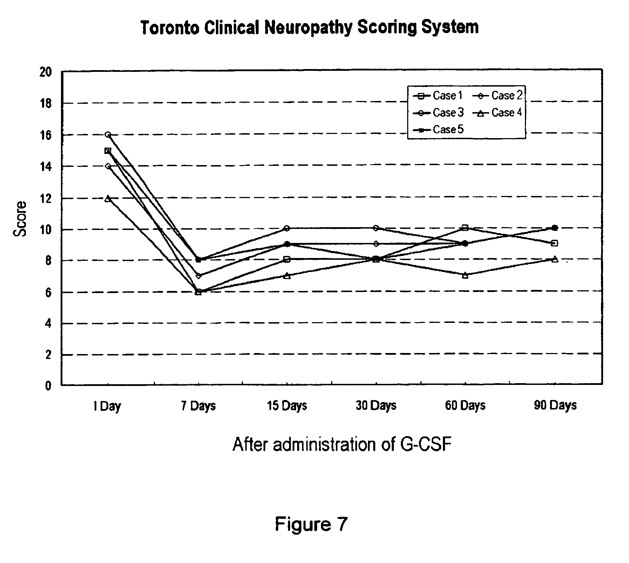

After 1 week, 15 days, 1 month, 2 months, and 3 months from the G-CSF administration, hematological and neurological tests were conducted by visits to the hospital to measure nerve conduction velocity (NCV) and the degree of diabetic peripheral neuropathy of the subject patients was measured by Toronto Clinical Neuropathy Scoring, and the overall test results were shown in Table 5, 7 and FIG. 7.

### A: Nerve Conduction Velocity

**[Table 5]**

| Nerve Conduction Velocity (m/s) | | | | | | |
|---|---|---|---|---|---|---|
| Classification | Day 0 | Day 7 | Day 15 | Day 30 | Day 60 | Day 90 |
| case 1 | 32.4 | 34.1 | 36.7 | 38.3 | 40.1 | 40.8 |
| case 2 | 31.6 | 32.5 | 34.1 | 40.1 | 41.3 | 42.6 |
| case 3 | 34.3 | 34.2 | 35.8 | 39.1 | 39.7 | 39.8 |
| case 4 | 30.8 | 32.9 | 33.7 | 39.2 | 41.6 | 41.2 |
| case 5 | 35.2 | 34.8 | 36.3 | 42.8 | 42.7 | 41.2 |

As seen from Table 5, it was confirmed that nerve conduction velocity improved until 90 days after the administration of G-CSF.

### B: Evaluation on Degree of Diabetic Peripheral Neuropathy

The present evaluation was performed by rating the subjects listed in Toronto Clinical Neuropathy Scoring System shown in Table 6. At this time, the evaluation results are presented by the total scores of each subject rating, in which 19 points is the maximum score and 0 point is the minimum score. Here, the higher the scores, the degree of diabetic peripheral neuropathy is more serious. The obtained results are presented in Table 7 and FIG. 7.

**[Table 6]**

| Toronto Clinical Neuropathy Scoring System Evaluation | |
|---|---|
| Classification | Toronto Score |
| Foot (Total of 6 points, 1 point for each subject) | Pain |
| | Numbness |
| | Tingling |
| | Weakness |
| | Ataxia |
| | Upper-limb symptoms |
| Reflex scores (Total of 8 points, 2 point for each subject) | Right knee |
| | Left knee |
| | Right ankle |
| | Left ankle |
| | Pinprick |
| Sensory test scores (Total of 5 points, 1 point for each subject) | Temperature |
| | Light-Touch |
| | Vibration |
| | Position |
| Total Score | 19 points |

**[Table 7]**

| Results of Toronto Clinical Neuropathy Scoring System Evaluation | | | | | | |
|---|---|---|---|---|---|---|
| Classification | Score | | | | | |
| | Day 0 | Day 7 | Day 15 | Day 30 | Day 60 | Day 90 |
| case 1 | 15 | 6 | 8 | 8 | 10 | 9 |
| case 2 | 14 | 7 | 9 | 9 | 9 | 10 |
| case 3 | 16 | 8 | 10 | 10 | 9 | 10 |
| case 4 | 12 | 6 | 7 | 8 | 7 | 8 |
| case 5 | 15 | 8 | 9 | 8 | 9 | 10 |

As seen from Table 7 and FIG. 7, Toronto Clinical Neuropathy Scoring System evaluation scores improved until 90 days after the administration of G-CSF. Thus, the therapeutic effects of the therapeutic agent according to the present invention on diabetic peripheral neuropathy were clinically confirmed.

Accordingly, it is known that the G-CSF can be useful for preventing and treating diabetic peripheral neuropathy.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. G-CSF (Granulocyte-Colony Stimulating Factor) for use in a method of preventing or treating diabetic peripheral neuropathy.

2. The G-CSF for use according to claim 1, wherein the G-CSF is obtained and separated from natural or recombinant origin.

3. The G-CSF for use according to claim 1, wherein the G-CSF is a recombinant human granulocyte colony-stimulating factor (rhG-CSF).

## Patentansprüche

1. G-CSF (Granulozyten-Kolonie stimulierender Faktor) zur Verwendung in einem Verfahren zur Prävention oder Behandlung diabetischer peripherer Neuropathie.

2. G-CSF zur Verwendung gemäß Anspruch 1, wobei der G-CSF aus natürlicher oder rekombinanter Provenienz erhalten und abgetrennt ist.

3. G-CSF zur Verwendung gemäß Anspruch 1, wobei der G-CSF ein rekombinanter humaner Granulozyten-Kolonie stimulierender Faktor (rhG-CSF) ist.

## Revendications

1. G-CSF (facteur stimulant les colonies de granulocytes) pour l'utilisation dans une méthode de prévention et de traitement de la neuropathie périphérique diabétique.

2. G-CSF pour l'utilisation selon la revendication 1, ledit G-CSF étant obtenu et séparé d'origine naturelle ou recombinante.

3. G-CSF pour l'utilisation selon la revendication 1, ledit G-CSF étant un facteur humain recombinant stimulant les colonies de granulocytes (rhG-CSF).
